# EUROPÄISCHE PATENTANMELDUNG

(11) **EP 2 082 709 A1**
(43) Veröffentlichungstag der Anmeldung: **29.07.2009**
(21) Anmeldenummer: 09000844.2
(22) Anmeldetag: 22.01.2009
(51) Int. Cl.: A61F 5/01

(54) **Orthopädische Sprunggelenkstütze**

(30) Priorität: 24.01.2008 DE 202008001405 U
(71) Anmelder: Bort GmbH, 71384 Weinstadt (DE)
(72) Erfinder: Bort, Wolfgang, 71384 Weinstadt (DE)
(74) Vertreter: Patentanwälte Ruff, Wilhelm, Beier, Dauster & Partner

(57) **Zusammenfassung**

Die Erfindung betrifft eine orthopädische Sprunggelenkstütze mit einem flexiblen Strumpfabschnitt, der einen Fußbereich und einen Beinbereich umfasst, wobei an dem Beinbereich ein medialer und ein lateraler Stabilisierungsbereich angeordnet sind, und wobei dem Fußbereich wenigstens ein den Fußbereich plantar umspannendes Stützband zugeordnet ist, das zu seinem freien Ende hin in einen Zugbandabschnitt fortgesetzt ist, der Befestigungsmittel zum lösbaren, gespannten Festlegen an einem Befestigungsbereich des Strumpfabschnittes oder eines Stabilisierungsbereiches aufweist. Erfindungsgemäß sind zwei einander plantar überlappende, sich kreuzende Stützbänder (7,9) vorgesehen, von denen eines medial und das andere lateral an jeweils einem Endbereich mit dem Strumpfabschnitt (2,3) verbunden sind, und die mittels ihrer Zugbandabschnitte (8,10) im Beinbereich (3) des Strumpfabschnittes festlegbar sind.

## Beschreibung

Die Erfindung betrifft eine orthopädische Sprunggelenkstütze mit einem flexiblen Strumpfabschnitt, der einen Fußbereich und einen Beinbereich umfasst, wobei an dem Beinbereich ein medialer und ein lateraler Stabilisierungsbereich angeordnet sind, und wobei dem Fußbereich wenigstens ein den Fußbereich plantar umspannendes Stützband zugeordnet ist, das zu seinem freien Ende hin in einen Zugbandabschnitt fortgesetzt ist, der Befestigungsmittel zum lösbaren, gespannten Festlegen an einem Befestigungsbereich des Strumpfabschnittes oder eines Stabilisierungsbereiches aufweist.

Eine derartige Sprunggelenkstütze ist aus der DE 600 17 944 T2 bekannt. Die Sprunggelenkstütze weist einen elastisch flexiblen Strumpfabschnitt auf, der nach Art einer Manschette einen Fuß und einen unteren Beinabschnitt eines Patienten umgibt. Der Strumpfabschnitt weist sowohl lateral als auch medial jeweils einen formstabilen Stabilisierungsbereich auf, die fest mit dem Strumpfabschnitt verbunden sind. An dem Fußbereich des Strumpfabschnittes ist ein den Fußbereich umschlingendes und damit auch plantar um den Fußbereich herumgeführtes Stützband befestigt. Zu seinem freien Ende hin ist das Stützband in einen schmaleren Zugbandabschnitt fortgesetzt, der mittels eines Klettverschlusses an dem lateralen Stabilisierungsbereich des Beinbereiches lösbar und unter Spannung festlegbar ist.

Aufgabe der Erfindung ist es, eine orthopädische Sprunggelenkstütze der eingangs genannten Art zu schaffen, die ein verbessertes Anlegen und eine verbesserte Stützung eines menschlichen Sprunggelenkes ermöglicht.

Diese Aufgabe wird dadurch gelöst, dass zwei einander plantar überlappende, sich kreuzende Stützbänder vorgesehen sind, von denen eines medial und das andere lateral an jeweils einem Endbereich mit dem Strumpfabschnitt verbunden sind, und die mittels ihrer Zugbandabschnitte im Beinbereich des Strumpfabschnittes festlegbar sind. Die Stützbänder sind somit an ihren Endbereichen auf gegenüberliegenden Seiten des Strumpfabschnittes festgelegt, nämlich medial und lateral. Beide Stützbänder werden entgegengesetzt zueinander und damit sich kreuzend sowie sich plantar im Fußbereich überlappend plantar um den Fußbereich herumgeführt und anschließend im Beinbereich des Strumpfabschnittes mit den zugehörigen Zugbandabschnitten lösbar unter Spannung befestigt. Vorzugsweise überkreuzen sich die Zugbandabschnitte frontal im distalen Bereich des Beinbereiches des Strumpfabschnittes erneut, um in entgegengesetzten Richtungen um den Beinbereich herumgeführt zu werden. Dadurch, dass die Stützbänder auf gegenüberliegenden Seiten des Fußbereiches angreifen und einander überlappend in entgegengesetzten Richtungen plantar um den Fußbereich herumgeführt sind, führen Zugbelastungen auf die beiden Stützbänder zu einer gleichmäßigen Belastung beider Seiten des Fußbereiches, d.h. sowohl der lateralen als auch der medialen Seite. Je nach gewünschter Stützung kann zudem individuell die laterale oder mediale Seite mit größerer Spannung versehen werden, so dass je nach Indikation eine laterale oder mediale Verlagerung der gewünschten Stützfunktion erzielbar ist. Die beiden voneinander unabhängigen Stützbänder ermöglichen insoweit eine individuelle Anpassung. Die Breite der Stützbänder ist auf die zu stützende, plantare Länge des Fußbereiches abgestimmt. Die Stützbänder selbst sind demzufolge wesentlich breiter als die anschließenden Zugbandabschnitte. Vorzugsweise sind als Befestigungsmittel im Bereich der Zugbandabschnitte Klettverschlusselemente vorgesehen. Es ist vorzugsweise vorgesehen, jeden Zugbandabschnitt innen- oder außenseitig mit den zueinander korrespondierenden Klettverschlusselementen zu versehen, so dass der Zugbandabschnitt durch entsprechendes Umlegen im Bereich einer Befestigungsschlaufe an sich selbst fixierbar ist. Die beiden Stabilisierungsbereiche sind vorzugsweise mit formstabilen Stützeinlagen versehen, die mit den entsprechend medialen und lateralen Bereichen des manschettenartigen Strumpfabschnittes vernäht oder in anderer Art und Weise an diesem fixiert sind.

In Ausgestaltung der Erfindung ist dem Beinbereich des Strumpfabschnittes eine separate, längenveränderbare Ringschlaufe zugeordnet, die zur Fixierung des Beinbereichs an einem proximalen Sprunggelenksbereich eines Patienten beiträgt. Die Ringschlaufe ist im proximalen Bereich des Beinbereiches des Strumpfabschnittes angeordnet und vorzugsweise als separates Zugband ausgeführt, das durch Befestigungsmittel, vorzugsweise in Form von Klettverschlusselementen, lösbar und längenveränderbar fixierbar ist. Vorzugsweise wird die Ringschlaufe somit durch ein offenes Zugband gebildet, das in angelegtem Zustand der Sprunggelenkstütze ringförmig fixierbar ist. Dadurch ist ein einfacher Ein- oder Ausstieg für einen Patienten ermöglicht. Das Fixieren der Ringschlaufe gewährleistet die definierte Positionierung der lateralen und medialen Stabilisierungsbereiche im proximalen Bereich des Beinbereiches der Stütze. Die beschriebene, separate und längenveränderbare Ringschlaufe kann auch für orthopädische Sprunggelenkstützen vorgesehen sein, die - wie eingangs beschrieben - ausgeführt sind. Eine zusätzliche plantare Überlappung von zwei sich kreuzenden Stützbändern im Fußbereich muss bei dieser Ausführung nicht notwendigerweise vorgesehen sein.

In weiterer Ausgestaltung der Erfindung ist im Beinbereich des Strumpfabschnittes eine im angelegten Zustand der Sprunggelenkstütze in Umfangsrichtung begrenzt verlagerbare Befestigungsschnalle vorgesehen, die mit in Umfangsrichtung einander gegenüberliegenden Umlenkbügeln für beide Zugbandabschnitte beider Stützbänder versehen ist. Die Befestigungsschnalle ist somit in Umfangsrichtung schwimmend gelagert. Vorteilhaft sind an der Befestigungsschnalle beide Zugbandabschnitte und damit auch beide Stützbänder fixierbar. Der eine Zugbandabschnitt bildet im befestigten Zustand jeweils die Gegenkraft und damit den Gegenhalter für den anderen Zugbandabschnitt. Die Befestigungsschnalle ist somit nach Festlegung beider Zugbandabschnitte in ihrer Position fixiert. Die begrenzte Verlagerbarkeit der Befestigungsschnalle gewährleistet Ausgleichsmöglichkeiten.

In weiterer Ausgestaltung der Erfindung weist der Beinbereich des Strumpfabschnittes einen dorsalen Verbindungssteg zwischen dem lateralen und dem medialen Stabilisierungsbereich auf. Dieser dorsale Verbindungssteg ist einstückiger Teil des Strumpfabschnittes oder in anderer Art und Weise unlösbar mit dem medialen und dem lateralen Bereich des Strumpfabschnittes und/oder dem medialen und lateralen Stabilisierungsbereich verbunden.

Weitere Vorteile und Merkmale der Erfindung ergeben sich aus der nachfolgenden Beschreibung einer bevorzugten Ausführungsform der Erfindung, die anhand der Zeichnungen dargestellt ist.
- Fig. 1: zeigt perspektivisch und schematisch eine bevorzugte Ausfüh- rungsform einer erfindungsgemäßen Sprunggelenkstütze in angelegtem Zustand,
- Fig. 2: die Sprunggelenkstütze nach Fig. 1 in teilweise geöffnetem Zu- stand,
- Fig. 3: eine plantare Ansicht der Sprunggelenkstütze nach den Fig. 1 und 2 und
- Fig. 4: in vergrößerter Darstellung einen Ausschnitt der Sprungge- lenkstütze nach den Fig. 1 bis 3 auf Höhe eines lateralen Stabi- lisierungsbereiches der Sprunggelenkstütze.

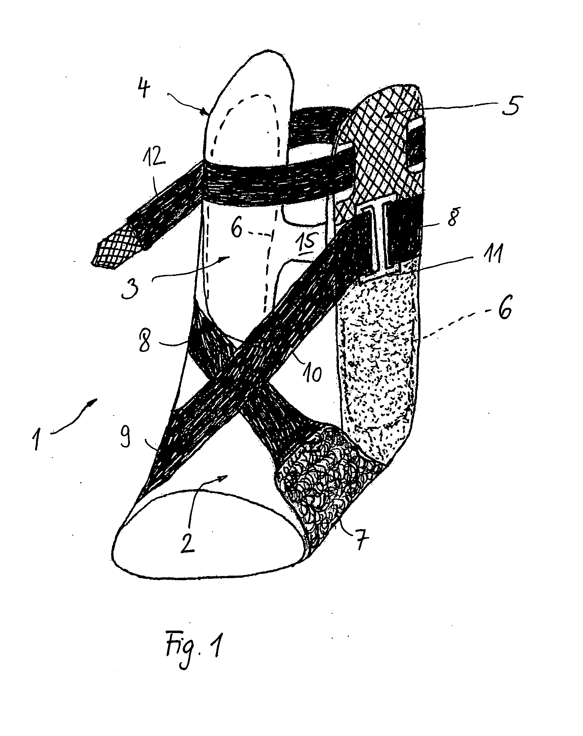

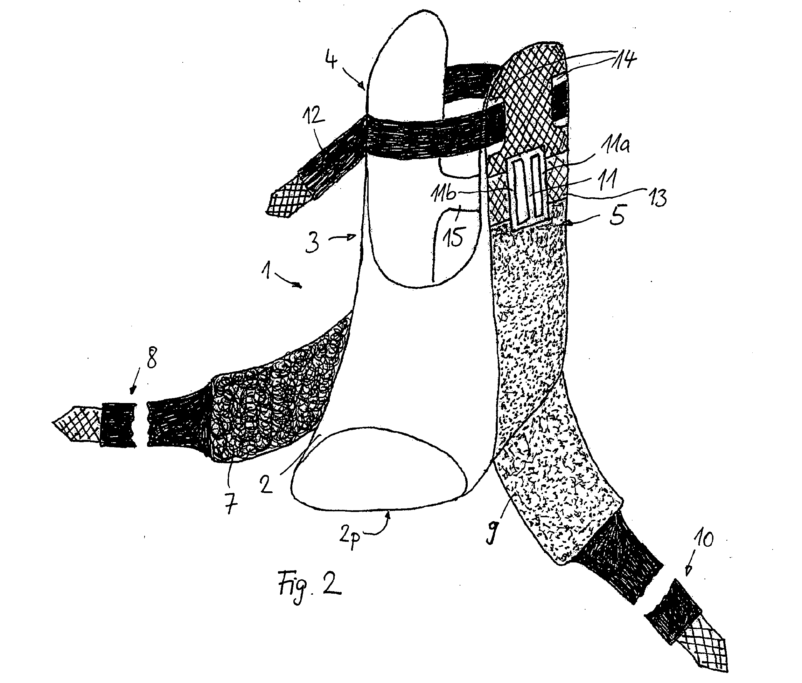

Eine Sprunggelenkstütze 1 nach den Fig. 1 bis 4 weist einen manschettenartigen Strumpfabschnitt auf, der einen Fußbereich 2 und einen Beinbereich 3 umfasst. Der Strumpfabschnitt ist aus flexiblem, elastisch nachgiebigem Textilmaterial, wie insbesondere einem Gewirke oder Gestricke, hergestellt. Der Fußbereich 2 bildet einen in Umfangsrichtung geschlossenen Hüllkörper. Zu einem Zehenbereich eines menschlichen Fußes ist der Fußbereich 2 offen. Der Beinbereich 3 ist aus dem Fußbereich 2 einstückig fortgesetzt und weist zwei einander gegenüberliegende, medial und lateral angeordnete Seitenbereiche auf, die als Stabilisierungsbereiche 4, 5 ausgeführt sind. Hierzu ist jeder Stabilisierungsbereich 4, 5 zumindest weitgehend mit einer formstabilen Stützeinlage 6 versehen, die in Fig. 1 angedeutet ist. Frontal ist der Beinbereich offen, wie anhand der Fig. 1 und 2 erkennbar ist. Dorsal ist der Beinbereich 3 teilweise geschlossen. Hierzu weist der Strumpfabschnitt einen Verbindungssteg 15 auf, der einstückiger Teil des Strumpfabschnittes ist und demzufolge aus dem Material des Strumpfabschnittes hergestellt ist. Der Verbindungssteg 15 verbindet dorsal den medialen und den lateralen Seitenbereich des Strumpfabschnittes miteinander. Damit werden durch den Verbindungssteg 15 auch die lateralen und medialen Stabilisierungsbereiche 4, 5 der Sprunggelenkstütze 1 miteinander verbunden. Der Verbindungssteg 15 kann relativ breit gestaltet sein. Distal schließt an den Verbindungssteg ein offener Abschnitt für einen Fersenbereich eines menschlichen Fußes an. Proximal zum Verbindungssteg 15 ist der Beinbereich 3 ebenfalls offen.

An den proximalen Seitenbereichen des Strumpfabschnittes, nämlich medial und lateral, ist jeweils eine Durchtrittsschlaufe 14 vorgesehen, durch die ein Zugband 12 hindurchgeschlauft ist. Das Zugband 12 ist ein offenes Zugband, das mit dem Strumpfabschnitt entweder gar nicht oder lediglich an einem der beiden Seitenbereiche fest verbunden ist. Es ist als ringförmige Schlaufe zusammenfügbar, indem ein freies Ende des Zugbandes 12 mit Klettverschlusselementen versehen ist, die auf korrespondierend gestalteten Klettverschlusselementen an einer Außenseite des Zugbandes 12 festlegbar sind. Das Zugband 12 weist somit je nach Gestaltung innen- und/oder außenseitig an entsprechend geeigneten Stellen Klettverschlusselemente auf, die eine lösbare Fixierung des Zugbandes in unterschiedlichen Längen und in ringförmiger Schlaufe ermöglichen. Die Durchtrittsschlaufe 14 dient als Verliersicherung für das Zugband 12. Falls an beiden Seitenbereichen des Beinbereiches 3 des Strumpfabschnittes jeweils eine Durchtrittsschlaufe 14 vorgesehen ist, können beide Durchtrittsschlaufen als Verliersicherungen für das Zugband vorgesehen sein. Das Zugband 12 ist durch die Durchtrittsschlaufen 14 hindurchgeschlauft und kann aus diesen herausgezogen oder erneut in diese eingefädelt werden. Das Zugband 12 dient als geschlossene Ringschlaufe in angelegtem Zustand der Sprunggelenkstütze 1 zur Fixierung des proximalen Bereiches der Sprunggelenkstütze 1 an einem entsprechenden Bein eines Patienten. Für das Anlegen oder Ausziehen der Sprunggelenkstütze 1 wird das Zugband 12 geöffnet. Nach dem Anlegen der Sprunggelenkstütze 1 wird das Zugband 12 um das Bein herumgespannt und fixiert. Dadurch ist eine sichere und stabile Positionierung der Stabilisierungsbereiche 4, 5 ermöglicht.

In distaler Verlängerung der Stabilisierungsbereiche 4, 5 ist an dem Strumpfabschnitt sowohl medial als auch lateral jeweils ein Stützband 7, 9 fixiert. Beim dargestellten Ausführungsbeispiel wird jedes Stützband 7, 9 durch Verlängerung eines Textilstreifens gebildet, der eine Außenfläche des jeweiligen Stabilisierungsbereiches 4, 5 bildet. Der Textilstreifen ist bis in den Fußbereich 2 insbesondere durch Vernähen fest mit dem Strumpfabschnitt verbunden. Am Fußbereich 2 des Strumpfabschnittes ragt der Textilstreifen dann frei ab und bildet das eigentliche Stützband 7, 9. Die Breite jedes Stützbandes 7, 9 entspricht der zu stützenden plantaren Länge des Fußbereiches 2. Die Plantarseite des Fußbereiches 2 wird mit dem Bezugszeichen 2p bezeichnet. Die Länge jedes Stützbandes 7, 9 - in Zugrichtung gesehen - ist so gewählt, dass jedes Stützband 7, 9 quer über die Plantarseite 2p des Fußbereiches erstreckt und auf der gegenüberliegenden Seite wieder um den Fußbereich 2 herum zu dem Beinbereich 3 des Strumpfabschnittes geführt werden kann. An das jeweilige freie Ende jedes Stützbandes 7, 9 schließt ein Zugbandabschnitt 8, 10 an, der gegenüber der Breite des Stützbandes 7, 9 schmaler ausgeführt ist. Die Zugbandabschnitte 8, 10 weisen eine Länge auf, die es ihnen ermöglicht, bis in den proximalen Bereich des Beinbereiches 3 geführt zu werden und dort fixiert zu werden.

Beim dargestellten Ausführungsbeispiel sind die Zugbandabschnitte mit Befestigungsmitteln in Form von Klettverschlusselementen versehen, wobei zueinander korrespondierende Klettverschlusselemente innen-und/oder außenseitig am jeweiligen Zugbandabschnitt 8, 10 vorgesehen sind. Dadurch ist es möglich, den jeweiligen Zugbandabschnitt 8, 10 an einer entsprechenden Befestigungsstelle umzuschlaufen und an sich selbst zu fixieren.

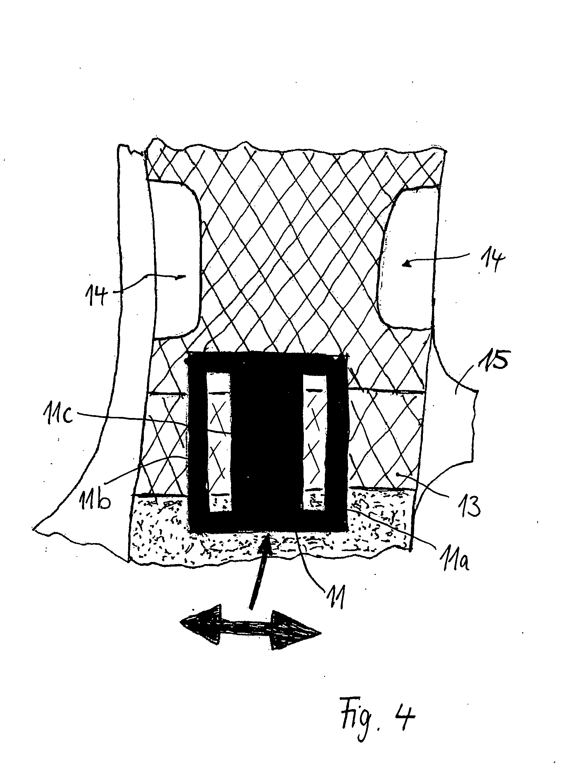

Zur Fixierung der Zugbandabschnitte 8, 10 am Beinbereich 3 des Strumpfabschnittes ist dem lateralen Stabilisierungsbereich 5 der Sprunggelenkstütze 1 eine Befestigungsschnalle 11 zugeordnet, die mit zwei Umlenkbügeln 11a, 11 b versehen ist, die an gegenüberliegenden Seiten der Befestigungsschnalle 11 vorgesehen sind. Die Befestigungsschnalle 11 selbst ist mittels eines Führungssteges 11 c längs eines Führungsbandabschnittes 13 in Richtung des Doppelpfeiles (Fig. 4) begrenzt in Umfangsrichtung des Beinbereiches 3 translatorisch verlagerbar gehalten. Das Führungsband 13 bildet einen über die Breite des Stabilisierungsbereiches 5 erstreckten Bandabschnitt, der an seinen Endbereichen mit dem Stabilisierungsbereich 5 bzw. mit dem Strumpfabschnitt fest verbunden ist. Der übrige Teil des Bandabschnittes 13 liegt ohne Verbindung lediglich auf der Außenfläche des Stabilisierungsbereiches 5 auf, so dass die Befestigungsschnalle 11 mittels ihres Führungssteges 11 c längs des Bandabschnittes verschiebbar ist. Jeweils ein Zugbandabschnitt 8, 10 wird für eine Fixierung der Stützbänder 7, 9 am Fußbereich 2 durch den jeweils zugeordneten Umlenkbügel 11a, 11 b durchgeschlauft und anschließend durch Festzurren gespannt. Da beide Zugbandabschnitte die Befestigungsschnalle 11 in entgegengesetzten Richtungen mit Zugkraft beaufschlagen, wird die Befestigungsschnalle 11 zwangsläufig in einer entsprechenden Position fixiert.

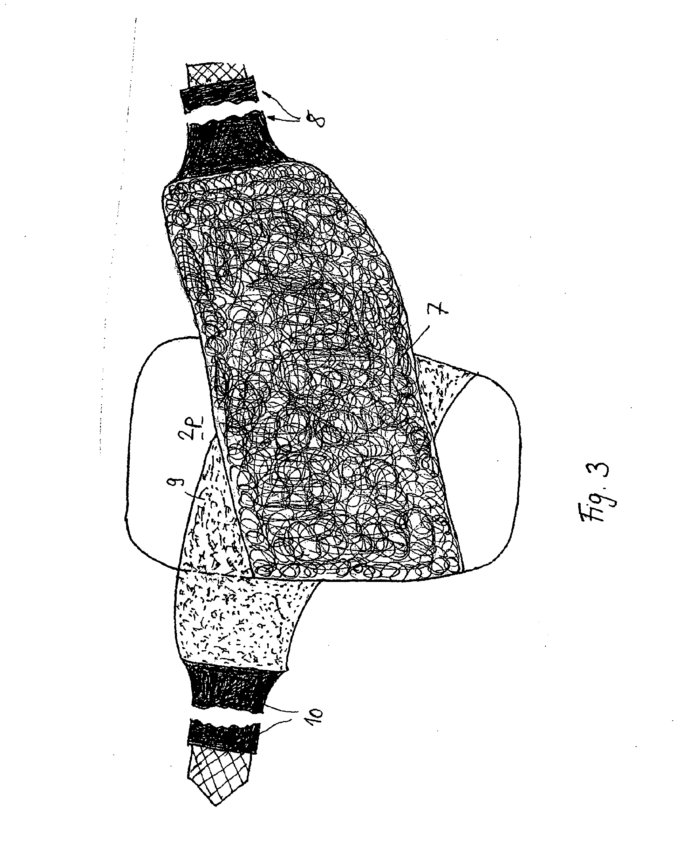

Um die Sprunggelenkstütze 1 anlegen zu können, wird aus einem Zustand der Sprunggelenkstütze 1 gemäß Fig. 2 zunächst das proximale Zugband 12 gelöst. Anschließend wird die Sprunggelenkstütze 1 mittels des Strumpfabschnittes über das menschliche Sprunggelenk gezogen, bis ein Vorderfußbereich des menschlichen Fußes nach vorne aus dem Fußbereich 2 des Strumpfabschnittes herausragt und ein Fersenbereich des menschlichen Fußes unterhalb des Verbindungssteges 15 gelangt ist. Nun wird das Zugband 12 gespannt, so dass die Stabilisierungsbereiche 4, 5 medial und lateral stützend am menschlichen Sprunggelenk fixiert sind. Dann werden die beiden Stützbänder 7, 9 von der lateralen bzw. der medialen Seite aus einander überkreuzend und einander überlappend plantar und im Fußbereich 2 herumgeschlungen (Fig. 3) und anschließend gemäß Fig. 1 mit ihrem Zugbandabschnitt 8, 10 zum Beinbereich 3 des Strumpfabschnittes nach oben geführt. Dabei überkreuzen sich die Zugbandabschnitte 8, 10, wie anhand der Fig. 1 erkennbar ist. Der eine Zugbandabschnitt 10 wird an der Befestigungsschnalle 11 durch den frontalen Umlenkbügel 11 b durchgeschlauft. Die Befestigungsschlaufe 11 ist beim dargestellten Ausführungsbeispiel am lateralen Stabilisierungsbereich 5 der Sprunggelenkstütze 1 angeordnet. Der andere Zugbandabschnitt 8 wird um den medialen Stabilisierungsbereich 4 herum zur dorsalen Seite des Strumpfbandabschnittes geführt und von der dorsalen Seite aus zum lateralen Stabilisierungsbereich 6 geführt. Dort wird der Zugbandabschnitt 8 um den dorsalen Umlenkbügel 11a herumgelegt. Beide Zugbandabschnitte 8, 10 werden mittels entsprechender Klettverschlusselemente nach dem Durchschlaufen durch den entsprechenden Umlenkbügel 11a, 11 b jeweils auf sich selbst fixiert. In vorteilhafter Weise kann zunächst ein Zugbandabschnitt relativ lose fixiert werden. Die Spannung dieses Zugbandabschnittes und damit auch des zugehörigen Stützbandes erfolgt anschließend durch das Festzurren des gegenüberliegenden Zugbandabschnittes, da dieser die schwimmend gelagerte Befestigungsschnalle 11 in Umfangsrichtung mitbewegt und dadurch zwangsläufig den bereits an der Befestigungsschnalle 11 fixierten ersten Zugbandabschnitt mitspannt.

## Patentansprüche

1. Orthopädische Sprunggelenkstütze mit einem flexiblen Strumpfabschnitt, der einen Fußbereich und einen Beinbereich umfasst, wobei an dem Beinbereich ein medialer und ein lateraler Stabilisierungsbereich angeordnet sind, und wobei dem Fußbereich wenigstens ein den Fußbereich plantar umspannendes Stützband zugeordnet ist, das zu seinem freien Ende hin in einen Zugbandabschnitt fortgesetzt ist, der Befestigungsmittel zum lösbaren, gespannten Festlegen an einem Befestigungsbereich des Strumpfabschnittes oder eines Stabilisierungsbereiches aufweist, **dadurch gekennzeichnet, dass** zwei einander plantar überlappende, sich kreuzende Stützbänder (7, 9) vorgesehen sind, von denen eines medial und das andere lateral an jeweils einem Endbereich mit dem Strumpfabschnitt (2, 3) verbunden sind, und die mittels ihrer Zugbandabschnitte (8, 10) im Beinbereich (3) des Strumpfabschnittes festlegbar sind.

2. Orthopädische Sprunggelenkstütze nach dem Oberbegriff des Anspruchs 1 oder nach Anspruch 1, **dadurch gekennzeichnet, dass** dem Beinbereich (3) des Strumpfabschnittes eine separate, längenveränderbare Ringschlaufe (12) zugeordnet ist, die zur Fixierung des Beinbereiches an einem proximalen Sprunggelenksbereich eines Patienten beiträgt.

3. Orthopädische Sprunggelenkstütze nach Anspruch 1 oder 2, **dadurch gekennzeichnet, dass** im Beinbereich (3) des Strumpfabschnittes eine in angelegtem Zustand der Sprunggelenkstütze in Umfangsrichtung begrenzt verlagerbare Befestigungsschnalle (11) vorgesehen ist, die mit in Umfangsrichtung einander gegenüberliegenden Umlenkbügeln (11a, 11b) für beide Zugbandabschnitte (8, 10) beider Stützbänder (7, 9) versehen ist.

4. Orthopädische Sprunggelenkstütze nach Anspruch 1 oder 2, **dadurch gekennzeichnet, dass** der Beinbereich (3) des Strumpfabschnittes einen dorsalen Verbindungssteg (15) zwischen dem lateralen und dem medialen Stabilisierungsbereich (4, 5) aufweist.
